# EUROPÄISCHE PATENTSCHRIFT

(11) **EP 1 292 235 B1**
(45) Veröffentlichungstag und Bekanntmachung des Hinweises auf die Patenterteilung: **20.10.2004**
(21) Anmeldenummer: 00926634.7
(22) Anmeldetag: 31.05.2000
(51) Int. Cl.: A61B 17/15, A61B 19/00

(54) **VORRICHTUNG ZUR POSITIONIERUNG EINES CHIRURGISCHEN INSTRUMENTES**
DEVICE FOR POSITIONING A SURGICAL INSTRUMENT
DISPOSITIF POUR LE POSITIONNEMENT D'UN INSTRUMENT CHIRURGICAL

(43) Veröffentlichungstag der Anmeldung: 19.03.2003
(73) Patentinhaber: Stratec Medical AG, 4436 Oberdorf (CH)
(72) Erfinder: ZIRNGIBL, Nicolas, CH-4104 Oberwil (CH); MATHYS, Stefan, CH-4053 Basel (CH); HERZOG, Roland, CH-4437 Waldenburg (CH); UHRI, Patrick, CH-4410 Liestal (CH)
(74) Vertreter: Lusuardi, Werther Giovanni, Dr.
(86) Internationale Anmeldenummer: PCT/CH2000/000307
(87) Internationale Veröffentlichungsnummer: WO 2001/091647

(56) Entgegenhaltungen:
- EP-A- 0 604 697
- WO-A-99/59490
- CH-A- 608 874
- US-A- 4 938 762
- US-A- 5 383 454
- US-A- 5 454 810
- US-A- 5 891 144
- US-A- 5 961 527

## Beschreibung

Die Erfindung bezieht sich auf eine Vorrichtung zur Positionierung und/oder Führung von chirurgischen Instrumenten gemäss dem Oberbegriff des Patentanspruchs 1.

Eine solche Vorrichtung ist aus EP-A-604697 bekannt.

Im Falle chirurgischer Operationen ist zur Ausführung einer exakten Osteotomie oder Resektion von Knochen, beispielsweise bei einer Implantation einer Gelenk-Endoprothese, die mechanische Werkzeugführung mittels Schnittblöcken oder Bohrund Sägeschablonen häufig unabdingbar.

Eine Vorrichtung mit mehreren Präzisionsinstrumenten zur Durchführung von Sägeschnitten an Knochen, insbesondere des an das Kniegelenk grenzenden Femur und der Tibia ist aus der US 4,524,766 PETERSEN bekannt. Die verschiedenen Resektionsschnitte am Femur und an der Tibia werden bei dieser bekannten Vorrichtung mit verschiedenen Präzisionsinstrumenten, welche sequentiell am Femur oder an der Tibia befestigt werden, durchgeführt. Durch die Notwendigkeit, diese Präzisionsinstrumente an einem der Knochen zu befestigen, den geplanten Sägeschnitt auszuführen und das Präzisionsinstrument wieder zu entfernen bevor das nächste Präzisionsinstrument für einen weiteren Sägeschnitt an einem der Knochen befestigt werden kann, ergeben sich viele Operationsschritte.

Hier will die Erfindung Abhilfe schaffen. Der Erfindung liegt die Aufgabe zugrunde, eine Vorrichtung zu schaffen, mit welcher Werkzeugführungsschablonen einfach und mit präzisen Antriebselementen an einem Gelenk, insbesondere an einem Kniegelenk ausrichtbar und fixierbar sind. Zur erhöhten Stabilität soll durch die Erfindung bei Bedarf eine starre, mechanische Verbindung zwischen den beiden an das Gelenk grenzenden Knochen herstellbar sein. Ferner soll die Erfindung auch in Kombination mit einem chirurgischen Navigationssystem anwendbar sein.

Die Erfindung löst die gestellte Aufgabe mit einer Vorrichtung zur Positionierung und/oder Führung von chirurgischen Instrumenten, welche die Merkmale des Anspruchs 1 aufweist.

Die erfindungsgemässe Vorrichtung umfasst im wesentlichen einen externen Fixateur, welcher aus einem stabförmigen Längsträger mit einer Längsachse, zwei auf dem Längsträger parallel zur Längsachse verschiebbaren Backen und zwei quer zur Längsachse angeordneten und mittels der Backen am Längsträger fixierbaren Querträgern besteht. Jede Backe umfasst ein erstes Gelenk, mittels welchem der Querträger an einem seiner Enden um mindestens zwei senkrecht zueinander stehende Achsen drehbar mit der Backe verbunden ist. Am anderen, freien Ende des Querträgers sind Befestigungsmittel, insbesondere Knochenfixationsmittel anbringbar. Durch diese Befestigungsmittel an den Querträgern ist der externe Fixateur einerseits an einem oder beiden an das Gelenk angrenzenden Röhrenknochen oder andererseits an einem Rahmen befestigbar. Ferner umfasst die Vorrichtung einen auf dem Längsträger, vorzugsweise zwischen den Backen bezüglich der Längsachse axial verschiebbaren und arretierbaren Schlitten, woran ein Positionier- oder Führungselement zur Positionierung oder Führung von chirurgischen Instrumenten oder Werkzeugen anbringbar ist. Die Querträger umfassen mindestens ein ebenfalls um mindestens zwei Achsen drehbares, zweites Gelenk, wodurch der Längsträger bezüglich seiner durch die Befestigungsmittel gegebenen Fixationspunkte freier bewegbar, respektive einstellbar wird.

Eine externe Fixationsvorrichtung mit einem beidseitig teleskopierbaren Längsträger und zwei Backen, welche zwischen dem Längsträger und Knochenfixationsmitteln angeordnet sind, ist aus der WO 99/59489 MARTINELLI und der WO 99/59490 MARTINELLI bekannt. Durch die Backen sind die Knochenfixationsmittel, insbesondere Knochenschrauben relativ zum Längsträger um drei Achsen schwenkbar und mit diesem verbindbar. Für die Positionierung von chirurgischen Instrumenten oder Werkzeugen ergeben sich allerdings durch die Anordnung nur eines Gelenkes zwischen den Knochenfixationsmitteln und dem Längsträger zu wenig Freiheitsgrade für das ebenfalls mit dem Längsträger verbindbare Positionierelement.

In der bevorzugten Ausführungsform der erfindungsgemässen Vorrichtung umfasst diese ein quer zur Längsachse teleskopierbares Positionierelement, welches mit dem Schlitten verbindbar ist und somit auf dem Längsträger parallel zur Längsachse verschiebbar und fixierbar ist. Vorzugsweise besteht das Positionierelement aus einem Querstab mit einer Zentralachse, einem freien Ende zur Positionierung und/oder Führung von chirurgischen Instrumenten relativ zu einem Knochen und einem festen Ende, welches am Schlitten befestigt ist.

In einer anderen Ausführungsform umfasst jeder Querträger ein lösbar blockierbares, drittes Gelenk zwischen dem vorderen Ende und dem hinteren Ende, wodurch der Längsträger relativ zu seinen Fixationspunkten an den Knochen oder am Rahmen weitere Freiheitsgrade erhält, was eine exakte Positionierung respektive Führung des Positionier- respektive Führungselementes gestattet.

Ebenfalls möglich ist eine bezüglich quer zu Längsachse verlaufenden Teleskopachsen teleskopierbare Ausgestaltung der Querträger.

In einer weiteren Ausführungsform der erfindungsgemässen Vorrichtung sind am Positionierelement verschiedene werkzeugspezifische Führungsschablonen, insbesondere Schnittblöcke für die Durchführung von Osteotomie- oder Resektionsschnitten mittels einer chirurgischen Säge montierbar, so dass, beispielsweise bei den Resektionen am Femur und an der Tibia im Falle einer Implantation einer Knie-Endoprothese, sequentiell ein Schnittblock für die Schnittführung am Femur und ein Schnittblock für die Schnittführung an der Tibia einsetzbar ist.

Anstelle der sequentiellen Montage der Führungsschablonen ist je nach Ausgestaltung des Positionierelementes respektive der Führungsschablonen auch eine gleichzeitige Montage zweier oder mehrerer Führungsschablonen möglich.

In wiederum einer weiteren Ausführungsform der erfindungsgemässen Vorrichtung umfasst der Schlitten Antriebsmittel zur Drehung des Positionierelementes um die Zentralachse. Damit lassen sich beispielsweise bei Resektionen an Femur und Tibia im Falle einer Implantation einer Knie-Endoprothese die Steigungswinkel von nach posterior und/oder anterior abgeschrägten Schnittflächen einstellen.

In einer weiteren Ausführungsform der erfindungsgemässen Vorrichtung dient ein Antriebselement am Schlitten zur Drehung des Positionierelementes um eine Drehachse, welche senkrecht auf der Längsachse steht und senkrecht auf der Zentralachse steht, wodurch sich beispielsweise bei Resektionen an Femur und Tibia eine VarusNalgus-Korrektur einstellen lässt.

Die Antriebsmittel sowie das Antriebselement bestehen vorzugsweise aus Stellschrauben, mittels welcher das Positionierelement um zwei in den Schlitten integrierte Gelenke rotierbar ist.

In einer anderen Ausführungsform der erfindungsgemässen Vorrichtung sind die Backen je mit einem ersten Gelenk ausgestattet, welches eine Rotation der Querträger um drei senkrecht auf einander stehende Achsen gestattet. Dies lässt sich beispielsweise durch den Einsatz von Kugelgelenken erreichen. Durch diese Ausführung der Backen können die Knochenschrauben auch windschief eingedreht werden. Analog zu den ersten Gelenken können auch die zweiten und dritten Gelenke mehrere rotative Freiheitsgrade umfassen und beispielsweise als Kugelgelenke ausgestaltet sein.

Der Querträger und die Gelenke am Querträger, welche zur schwenkbaren Verbindung zwischen Backe und Querträger und je nach Ausführung der Querträger auch zur schwenkbaren Verbindung zwischen Querträger und Knochenfixationsmittel sowie zu einer Ausführung des Querträgers mit zwei durch ein Gelenk verbundenen Trägerteilen dienen, können so ausgestaltet sein, dass gleichzeitig alle sich an einem Querträger befindenden Gelenke durch ein einziges Feststellelement lösbar blockierbar sind. Eine solche Ausführung eines Dreigelenk-Statives wird in der CH 608 874 MEIER offenbart.

Der Längsträger bildet zusammen mit den fixierten Befestigungselementen eine Fixationsvorrichtung für die beiden an das Gelenk angrenzenden Knochen, wodurch das Gelenk blockiert werden kann und beide Knochen relativ zueinander gegen Translation und Rotation fixiert werden können.

Als Knochenfixationsmittel werden vorzugsweise Knochenschrauben eingesetzt.

In einer anderen Ausführungsform der erfindungsgemässen Vorrichtung sind die Führungsschalblonen mit Bohrungen zur Aufnahme von Knochenfixationsmitteln versehen, so dass jede Führungsschablone selbst am entsprechenden Knochen fixierbar ist. Damit ist der Vorteil erreichbar, dass der externe Fixateur nach der Fixation der Führungsschablone entfernt werden kann, wodurch der Chirurg bei der Durchführung der Schnitte keine Behinderung durch den Längsträger und die Befestigungselemente erhält. Ferner können in diesem Fall von Schnitten, diese bei einer beliebigen, für den Chirurgen optimalen Gelenkstellung (Extension, Flexion) ausgeführt werden.

Das Ausrichten der Führungsschablonen erfolgt in Falle der oben beschriebenen Ausführungsformen der erfindungsgemässen Vorrichtung manuell.

In einer weiteren Ausführungsform der erfindungsgemässen Vorrichtung ist diese mit einem chirurgischen Navigationssystem kombinierbar und umfasst Markierelemente, welche an den Knochenfixationsmitteln und an der Führungsschablone anbringbar sind. Vorzugsweise enthält jedes Markierelement mindestens drei nicht linear angeordnete, elektromagnetisch oder akustisch wirksame, aktive oder passive Marker.

Solche chirurgische Navigationssysteme mit integriertem Computer und Positionserfassungsvorrichtung zur Lagevermessung von im Raum bewegbaren chirurgischen Instrumenten und Geräten werden beispielsweise in der US 5, 383,454 BUCHHOLZ und der EP 0 359 773 SCHLÖNDORFF offenbart. Im Handel erhältlich sind chirurgische Navigationssysteme beispielsweise unter dem Namen "Surgigate" von der Firma MEDIVISION, Oberdorf, Schweiz. Üblicherweise umfassen solche Systeme auch Datenspeicher für Röntgenbilder oder ganze Computertomogramme (CT), welche zur Diagnose und Planung der Operation vor der Operation oder auch intraoperativ aufgenommen und im Datenspeicher des Computers abgespeichert werden können. Vielfach werden optoelektronische Positionserfassungsvorrichtungen eingesetzt, mittels welcher die Lage optischer, an den chirurgischen Instrumenten oder Geräten angebrachter Marker innerhalb eines dreidimensionalen Koordinatensystems im Operationsraum vermessbar ist. Optoelektronische Positionserfassungsvorrichtungen sind beispielsweise unter dem Namen Optotrak 3020 (Hersteller: Northern Digital, Ontario, Canada) im Hander erhältlich.

Als optische Marker werden üblicherweise LED's (Light Emitting Diodes) oder IRED's (Infrared Light Emitting Diodes) eingesetzt.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung die Arbeitsschritte bei einer chirurgischen Operation verringert werden können und damit die Operationszeit verkürzt wird. Speziell beim Einsatz in der Knieprothetik ergeben sich dadurch die folgenden Vorteile:
a) eine erhöhte Stabilität durch die Verbindung zwischen Femur und Tibia;
b) invasive Arbeitsschritte wie das Bohren eines intramedullären Kanals erübrigen sich;
c) nur ein Instrument für die Ausrichtung mehrerer Führungsschablonen notwendig;
d) Einstellmöglichkeit für eine Varus/Valgus-Korrektur; und
e) Einstellmöglichkeit für eine nach posterior ansteigende Schnittfläche (posterior slope).
   Wird die erfindungsgemässe Vorrichtung kombiniert mit einem chirurgischen Navigationssystem angewendet, lassen sich zusätzlich folgende Vorteile erzielen:
f) kein Ausrichten der Tibiaachse auf den 2. Strahl des Fusses;
g) Definition der mechanischen Beinachsen durch Digitalisieren der anatomischen Strukturen;
h) Positionserfassung aller Führungsschablonen; und
i) Möglichkeit der Messung von Auswirkungen verschiedener Weichteilkorrekturen (Soft tissue balancing).
   Werden zusätzlich präoperativ Röntgenaufnahmen erstellt, können folgende weitere Vorteile erreicht werden:
j) Graphische Bestimmung der Tibia-Achse;
k) Graphische Bestimmung des Hüftzentrums (im Falle eines zerstörten Gelenkes, das sich nicht mehr durch Pivotieren vermessen lässt); und
I) Beurteilung von Varus/Valgus-Fehlstellungen (graphische Erfassung), Möglichkeit zur Planung einer Korrektur der Beinstellung.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung einer Ausführungsform der erfindungsgemässen Vorrichtung;
Fig. 2 einen Ausschnitt aus einer Ausführungsform der erfindungsgemässen Vorrichtung;
Fig. 3 eine perspektivische Darstellung der in Fig. 2 dargestellten Ausführungsform der erfindungsgemässen Vorrichtung;
Fig. 4 ein Detail der in den Fig. 2 und 3 dargestellten Ausführungsform der erfindungsgemässen Vorrichtung;
Fig. 5 eine Ansicht eines Querträgers gemäss einer Ausführungsform der erfindungsgemässen Vorrichtung; und
Fig. 6 eine perspektivische Darstellung einer Ausführungsform der erfindungsgemässen Vorrichtung in Kombination mit einem chirurgischen Navigationssystem.

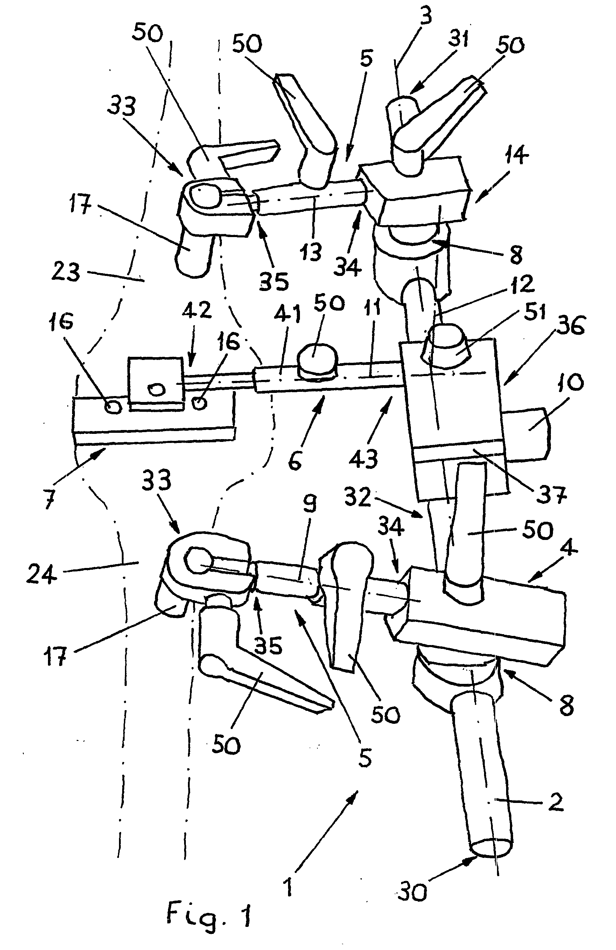

Fig. 1 zeigt eine Ausführungsform der erfindungsgemässen Vorrichtung, welche einen stabförmigen Längsträger 2 mit einer Längsachse 3, einem ersten Ende 30, einem zweiten Ende 31 und einem Mittelteil 32, zwei quer zur Längsachse 3 angeordnete Querträger 5 mit je einem hinteren Ende 34 und einem vorderen Ende 35, zwei auf dem Längsträger 2 parallel zur Längsachse 3 verschiebbare und lösbar fixierbare Backen 4;14 zur Verbindung der hinteren Enden 34 der Querträger 5 mit dem Längsträger 2 und einen auf dem Mittelteil 32 des Längsträgers 2 bezüglich der Längsachse 3 axial verschiebbaren und arretierbaren Schlitten 36 umfasst. Die Querträger 5 sind an den vorderen Enden 35 an einem, respektive zwei, an ein Gelenk angrenzenden Knochen, beispielsweise im Falle des Kniegelenkes am Femur 23 und an der Tibia 24 oder an einem Tragrahmen fixierbar, wobei der Tragrahmen am_Operationstisch oder an einem anderen Gestell beziehungsweise Gerät im Operationsraum angebracht sein kann. Jede Backe 4;14 umfasst ein lösbar blockierbares, erstes Gelenk 8, mittels welchem der mit der betreffenden Backe 4;14 verbundene Querträger 5 um drei senkrecht aufeinander stehende Achsen relativ zum Längsträger 2 rotierbar ist, wobei eine dieser drei Achsen mit der Längsachse 3 zusammenfällt. Ferner umfasst jeder Querträger 5 ein lösbar blockierbares, zweites Gelenk 33, wodurch die als Knochenschrauben ausgeführten Knochenfixationsmittel 17 schwenkbar mit den vorderen Enden 35 der Querträger 5 verbunden sind. Die zweiten Gelenke 33 sind in dieser Ausführungsform als Kugelgelenke ausgestaltet. Die Querträger 5 sind je entlang einer quer zur Längsachse 3 verlaufenden Teleskopachse 9;13 teleskopierbar und auf einer gewünschten Länge mittels Spannmitteln 50 lösbar blockierbar. Ebenfalls mittels Spannmitteln 50 blockierbar respektive lösbar sind die ersten und zweiten Gelenke 8;33. Mit dem Schlitten 36 verbunden ist ein Positionierelement 6, welches einen Querstab 41 mit einer Zentralachse 11, einem freien Ende 42 zur Positionierung und/oder Führung von chirurgischen Instrumenten relativ zu einem Knochen und einem festen Ende 43 aufweist, wobei der Querstab 41 an seinem festen Ende 43 mit dem Schlitten 36 verbunden ist. Der Querstab 41 ist koaxial zur Zentralachse 11 teleskopierbar und ebenfalls mittels eines Spannmittels 50 in einer gewünschten Länge lösbar blockierbar. Am freien Ende 42 des Positionierelementes 6 sind verschiedene Führungsschablonen 7 zur Führung von chirurgischen Instrumenten oder Werkzeugen anbringbar, wobei je nach Ausgestaltung der Führungsschablonen 7 gleichzeitig mehrere Führungsschablonen 7 anbringbar sind.

Der Schlitten 36 umfasst einen ersten Schraubenantrieb als Antriebsmittel 51 zur rotativen Bewegung des Querstabes 41 um die Zentralachse 11 und einen zweiten Schraubenantrieb als Antriebselement 10 zur rotativen Bewegung des Querstabes 41 um die Drehachse 12, welche senkrecht auf der Längsachse 3 und senkrecht auf der Zentralachse 11 steht.

An der Führungsschablone 7 sind Bohrungen 16 zur Aufnahme von Knochenschrauben angebracht, so dass die Führungsschablone 7 an die Tibia 24 oder an den Femur 23 schraubbar ist.

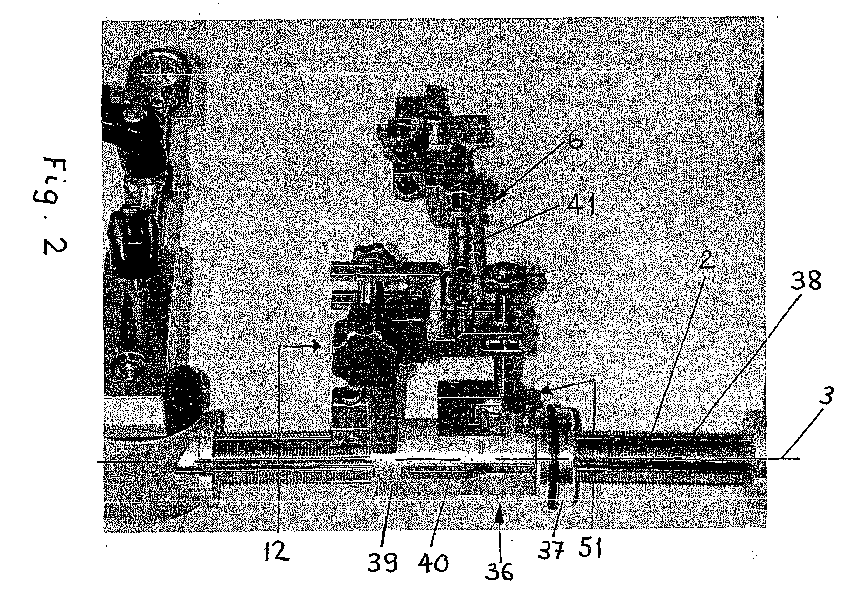

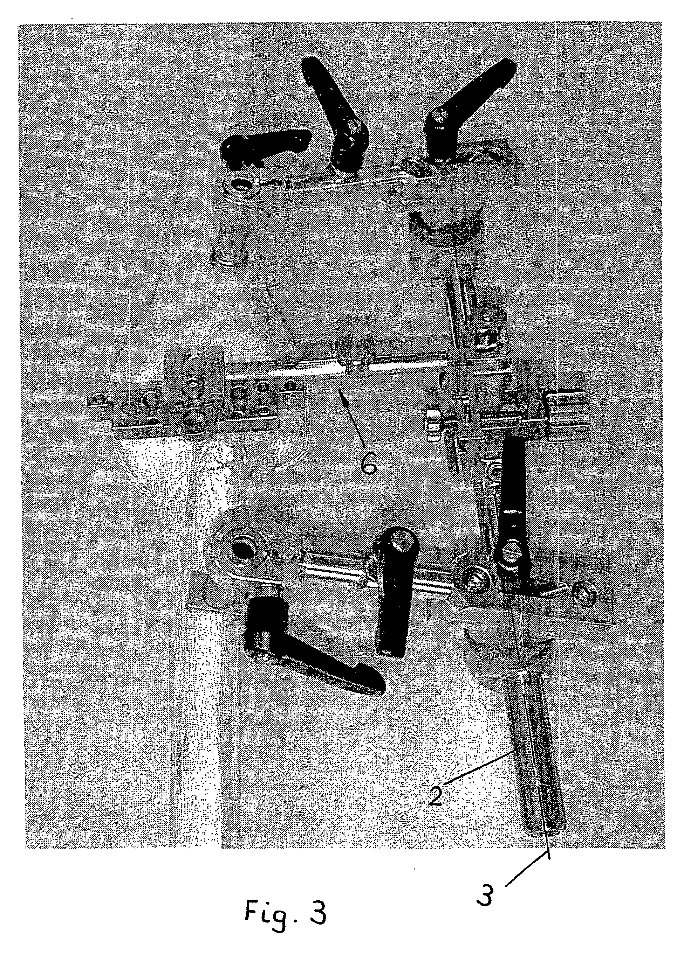

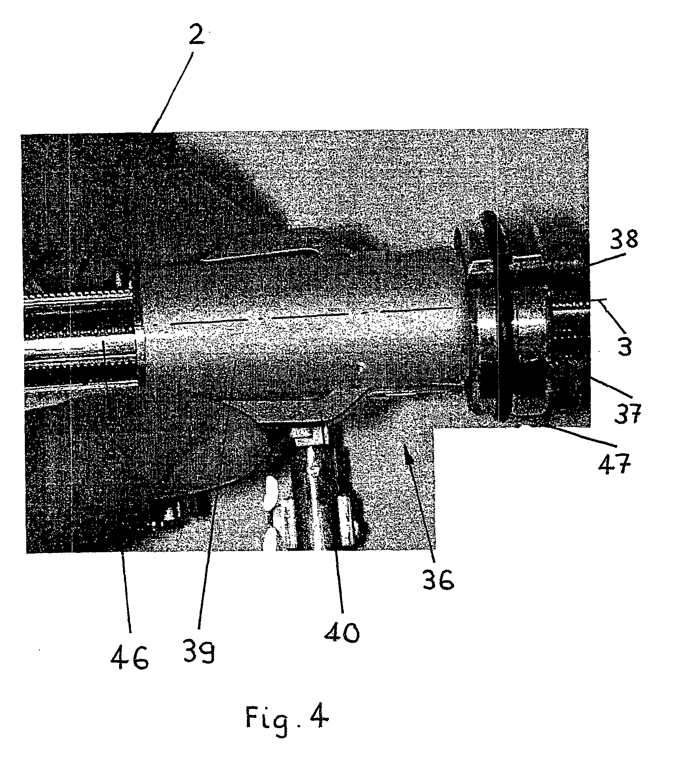

Fig. 2, 3 und 4 zeigen eine Ausführungsform der erfindungsgemässen Vorrichtung mit einem Längsträger 2, welcher ein Aussengewinde 38 und parallel zur Längsachse 3 mehrere Nuten 46 umfasst. Mittels dieser Nuten 46 und entsprechenden Nocken (nicht gezeichnet) am Schlitten 36 ist dieser gegen Rotation um die Längsachse 3 gesichert. Der Schlitten 36 weist auf dem Längsträger 2 ein- respektive ausklinkbare Positioniermittel 37 auf, so dass der Schlitten 36 bei ausgeklinkten Positioniermitteln 37 von Hand frei auf dem Längsträger 2 verschiebbar ist und bei eingeklinkten Positioniermitteln 37 formschlüssig und feineinstellbar auf dem Längsträger 2 verschiebbar ist. Dazu umfasst der Schlitten 36 eine auf dem Längsträger 2 parallel zur Längsachse 3 verschiebbare Buchse 39, damit verbunden die Positioniermittel 37, welche als axial teilbare Gewindebüchse ausgeführt sind, und in der Buchse 39 drehbar gelagerte Bedienungshebel 40. Die Gewindebüchse lässt sich durch Bedienen der Bedienungshebel 40 radial so weit öffnen, dass das Innengewinde der Gewindebüchse nicht mehr mit dem Aussengewinde 38 auf dem Längsträger 2 im Eingriff steht. Werden die Bedienungshebel 40 losgelassen, federt die Gewindebüchse durch die Wirkung der beispielsweise als Gummiring ausgeführten elastischen Mittel 47 radial zusammen, so dass das Innengewinde in der Gewindebüchse in das Aussengewinde 38 einrastet.

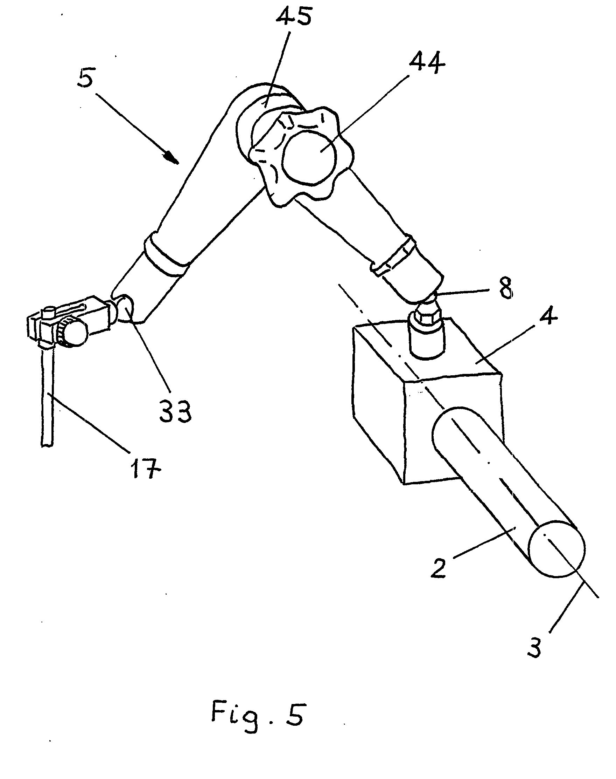

In Fig. 5 ist ein Querträger 5 einer Ausführungsform der erfindungsgemässen Vorrichtung dargestellt. Dieser Querträger 5 umfasst ein mittels eines Feststellelementes 44 lösbar blockierbares, drittes Gelenk 45. Der Querträger 5 ist mittels des ersten, als Kugelgelenk ausgeführten Gelenkes 8 schwenkbar mit der Backe 4 verbunden, während mittels des zweiten, ebenfalls als Kugelgelenk ausgeführten Gelenkes 33 das Knochenfixationsmittel 17 auch schwenkbar mit dem Querträger 5 verbunden ist. Die mittels des Feststellelementes 44 gleichzeitig blockierbare beziehungsweise lösbare Ausführung aller drei Gelenke 8;33;45 CH wird beispielsweise in der CH 608 874 MEIER dargestellt.

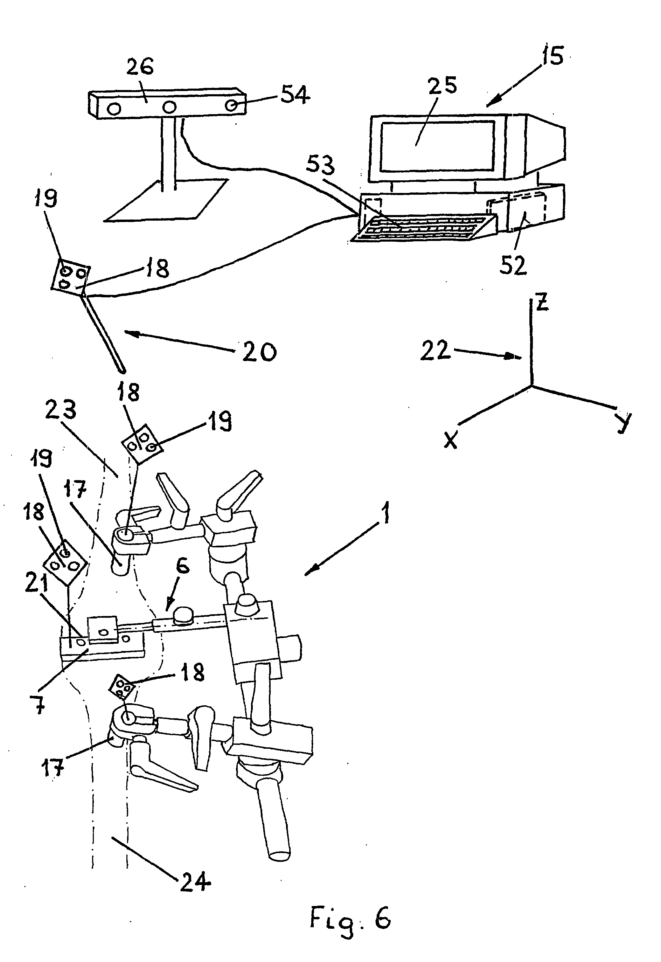

In Fig. 6 ist die bevorzugte Ausführungsform der erfindungsgemässen Vorrichtung in Kombination mit einem chirurgischen Navigationssystem dargestellt. Das chirurgische Navigationssystem umfasst einen integrierten Computer 15 mit Datenspeicher 52, Tastatur 53 und Bildschirm 25 und eine Positionserfassungsvorrichtung 26 mit drei Sensoren 54. Mittels der Positionserfassungsvorrichtung 26 erfolgt eine Lagevermessung der Marker 19, so dass durch den Computer 15 Position und Orientierung eines Pointers 20, der Knochenfixationsmittel 17 und der Führungsschablone 7, insbesondere der Schnittführungskante 21 bezüglich eines raumfesten Koordinatensystemes 22 ermittelt werden kann.

Die Anwendung der erfindungsgemässen Vorrichtung kombiniert mit einem chirurgischen Navigationssystem erfolgt beispielsweise bei den zur Implantation einer Knie-Endoprothese notwendigen Resektionsschnitten wie folgt:
- Befestigen je eines der als Knochenschrauben ausgeführten Knochenfixationsmittel 17 am Femur 23 und an der Tibia 24;
- Montage je eines mit drei nicht linear angeordneten Markern 19 versehenen Markierelementes an den Knochenfixationsmitteln 17;
- Abtasten der Anatomie, insbesondere der Kondylen am Femur 23 und der Tibiagelenkfläche mit dem Pointer 20 und Abspeicherung der abgetasteten Flächen im Datenspeicher 52 des Computers 15;
- Planung der auszuführenden Resektionsschnitte am Femur 23 und an der Tibia 24 am Computer 15, wobei die Resektionsschnitte am Bildschirm 25 an den gespeicherten Flächen eingezeichnet werden und deren Lage und Orientierung im raumfesten Koordinatensystem 22 berechnet wird;
- Anbringen des externen Fixateurs an den Knochenfixationsmitteln 17 und Montage des Positionierelementes 6 mit der oder den entsprechenden Führungsschablonen 7;
- Computergeführtes Einstellen des Positionierelementes 6 mit der oder den Führungsschalblonen 7. Die computergeführte Einstellung erfolgt durch Vergleichen der tatsächlichen, durch die Positionserfassungsvorrichung 26 gemessenen Position der Führungsschablone 7 mit der am Computer 15 geplanten Lage und Orientierung im raumfesten Koordinatensystem 22; und
- Ausführung der Resektionsschnitte mit dem entsprechenden chirurgischen Werkzeug.

## Patentansprüche

1. Vorrichtung zur Positionierung und/oder Führung von chirurgischen Instrumenten mit
A) einem stabförmigen Längsträger (2), welcher eine Längsachse (3), ein erstes Ende (30), ein zweites Ende (31) und einen Mittelteil (32) umfasst;
B) zwei quer zur Längsachse (3) angeordneten Querträgern (5), welche je ein hinteres Ende (34) und ein vorderes Ende (35) aufweisen und an den vorderen Enden (35) an einem respektive zwei Knochen oder an einem Tragrahmen fixierbar sind;
C) zwei auf dem Längsträger (2) parallel zur Längsachse (3) vetrschiebbaren und lösbar fixierbaren Backen (4;14) zur Verbindung der hinteren Enden (34) der Querträger (5) mit dem Längsträger (2); und
D) einem auf dem Längsträger (2) bezüglich der Längsachse (3) axial verschiebbaren und arretierbaren Schlitten (36), wobei
E) jede Backe (4;14) ein lösbar blockierbares, erstes Gelenk (8) umfasst, mittels welchem der jeweilige Querträger (5) um mindestens zwei senkrecht aufeinander stehende Achsen relativ zum Längsträger (2) bewegbar ist,
**dadurch gekennzeichnet, dass**
F) jeder Querträger (5) ein lösbar blockierbares, zweites Gelenk (33) umfarsst; und
G) der Schlitten (36) auf dem Längsträger (2) ein- respektive ausklinkbare Positioniermittel (37) aufweist, wodurch der Schlitten (36) bei ausgeklinkten Positioniermitteln (37) von Hand frei verschiebbar ist und bei eingeklinkten Positioniermitteln (37) formschlüssig und feineinstellbar auf dem Längsträger (2) verschiebbar ist.

2. Vorrichtung nach Anspruch 1, **dadurch gekennzeichnet, dass** die ersten Gelenke (8) drei rotative Freiheitsgrade aufweisen.

3. Vorrichtung nach Anspruch 1 oder 2, **dadurch gekennzeichnet, dass** die zweiten Gelenke (33) drei rotative Freiheitsgrade aufweisen.

4. Vorrichtung nach einem der Ansprüche 1 bis 3, **dadurch gekennzeichnet, dass** jeder Querträger (5) entlang einer quer zur Längsachse (3) verlaufenden Teleskopachse (9; 13) lösbar blockierbar teleskopierbar ist.

5. Vorrichtung nach einem der Ansprüche 1 bis 4, **dadurch gekennzeichnet, dass** jeder Querträger (5) ein lösbar blockierbares, drittes Gelenk (45) umfasst.

6. Vorrichtung nach Anspruch 5, **dadurch gekennzeichnet, dass** pro Querträger (5) die ersten, zweiten und dritten Gelenke (8;33;45) mittels eines Feststellelementes (44) lösbar blockierbar sind.

7. Vorrichtung nach Anspruch 6, **dadurch gekennzeichnet, dass** der Längsträger (2) ein Aussengewinde (38) aufweist und der Schlitten (36) eine auf dem Längsträger (2) parallel zur Längsachse (3) verschiebbare Buchse (39), als axial teilbare Gewindebüchse ausgestaltete Positioniermittel (37) und in der Buchse (39) drehbar gelagerte Bedienungshebel (40) umfasst, wobei die Gewindebüchse durch Bedienen der Bedienungshebel (40) radial so weit geöffnet wird, dass das Innengewinde der Gewindebüchse nicht mehr mit dem Aussengewinde (38) im Eingriff steht.

8. Vorrichtung nach einem der Ansprüche 1 bis 7, **dadurch gekennzeichnet, dass** sie ein Positionierelement (6) umfasst, welches einen Querstab (41) mit einer Zentralachse (11), einem freien Ende (42) zur Positionierung und/oder Führung von chirurgischen Instrumenten relativ zu einem Knochen und einem festen Ende (43) aufweist, wobei der Querstab (41) mit seinem festen Ende (43) mit dem Schlitten (36) verbindbar ist und koaxial zur Zentralachse (11) teleskopierbar ist.

9. Positioniervorrichtung nach Anspruch 8, **dadurch gekennzeichnet, dass** am freien Ende (42) des Positionierelementes (6) verschiedene Führungsschablonen (7) zur Führung von chirurgischen Instrumenten oder Werkzeugen anbringbar sind.

10. Positioniervorrichtung nach Anspruch 8 oder 9, **dadurch gekennzeichnet, dass** am Positionierelement (6) gleichzeitig mehrere Führungsschablonen (7) anbringbar sind.

11. Positioniervorrichtung nach einem der Ansprüche 8 bis 10, **dadurch gekennzeichnet, dass** der Schlitten (36) Antriebsmittel (51) umfasst, wodurch der Querstab (41) um die Zentralachse (11) rotierbar ist.

12. Positioniervorrichtung nach einem der Ansprüche 8 bis 11, **dadurch gekennzeichnet, dass** der Schlitten (36) ein Antriebselement (10) umfasst, wodurch der Querstab (41) um eine Drehachse (12), welche senkrecht auf der Längsachse (3) und senkrecht auf der Zentralachse (11) steht, rotierbar ist.

13. Positioniervorrichtung nach einem der Ansprüche 8 bis 12, **dadurch gekennzeichnet, dass** der Querstab (41) mittels Grob- und Feintrieben teteskopierbar ist.

14. Positioniervorrichtung nach einem der Ansprüche 11 bis 13, **dadurch gekennzeichnet, dass** die Antriebsmittel (51) Grob- und Feintriebe umfassen.

15. Positioniervorrichtung nach einem der Ansprüche 12 bis 14, **dadurch gekennzeichnet, dass** das Antriebselement (10) Grob- und Feintriebe umfasst.

16. Positioniervorrichtung nach einem der Ansprüche 13 bis 15, **dadurch gekennzeichnet, dass** die Grob- und Feintriebe stufenlos arbeiten.

17. Positioniervorrichtung nach einem der Ansprüche 13 bis 15, **dadurch gekennzeichnet, dass** die Grob- und Feintriebe gestuft arbeiten.

18. Positioniervorrichtung nach einem der Ansprüche 13 bis 17, **dadurch gekennzeichnet, dass** die Grob- und Feintriebe selbsthemmend ausgeführt sind.

19. Positioniervorrichtung nach einem der Ansprüche 13 bis 17, **dadurch gekennzeichnet, dass** die Grob- und Feintriebe arretierbar ausgeführt sind.

20. Positioniervorrichtung nach einem der Ansprüche 9 bis 19, **dadurch gekennzeichnet, dass** die Führungsschablonen (7) Bohrungen (16) zur Aufnahme von Knochenschrauben umfassen.

21. Vorrichtung nach einem der Ansprüche 1 bis 20, **dadurch gekennzeichnet, dass** sie Knochenfixationsmittel (17) umfasst, welche mit den vorderen Enden (35) verbindbar sind.

22. Vorrichtung nach Anspruch 21, **dadurch gekennzeichnet, dass** die Knochenfixationsmittel (17) mittels der zweiten Gelenke (33) drehbar mit je einem vorderen Ende (35) verbunden sind.

23. Positioniervorrichtung nach einem der Ansprüche 9 bis 22, **dadurch gekennzeichnet, dass** sie Markierelemente (18) umfasst, welche an der Führungsschablone (7) anbringbar sind.

24. Positioniervorrichtung nach einem der Ansprüche 21 bis 23, **dadurch gekennzeichnet, dass** sie Markierelemente (18) umfasst, welche an den Knochenfixationsmitteln (17) anbringbar sind.

25. Positioniervorrichtung nach Anspruch 23 oder 24, **dadurch gekennzeichnet, dass** jedes Markierelement (18) mindestens drei nicht linear angeordnete, elektromagnetisch oder akustisch wirksame Marker (19) umfasst, deren Position bezüglich eines raumfesten Koordinatensystems (22) durch eine elektronische Positionserfassungsvorrichtung (19) erfassbar ist.

## Claims

1. An apparatus for positioning and/or guiding surgical instruments, including
A) a rod-like, longitudinal carrier (2) which comprises a longitudinal axis (3), a first end portion (30), a second end portion (31), and a middle part (32);
B) two transverse couplers (5) which have each a rear end portion (34) and a front end portion (35) and which may be fastened with their front end portions (35) to one or two bones, respectively, or to a supporting frame;
C) two jaws (4; 14) displaceable parallel to the longitudinal axis (3) on the longitudinal carrier (2) and releasably lockable thereon for connecting the rear end portions (34) of the transverse couplers (5) with the longitudinal carrier (2), and
D) a slide member (36) axially displaceable relative to the longitudinal axis (3) on the longitudinal carrier (2) and lockable thereon,
E) each jaw (4; 14) comprising a releasably lockable, first joint (8) by means of which the corresponding transverse coupler (5) is movable relative to the longitudinal carrier (2) about at least two axes extending perpendicularly to each other,
**characterised in that**
F) each transverse coupler (5) comprises a releasably lockable, second joint (33); and
G) the slide member (36) is provided with positioning means (37) engageable and disengageable with the longitudinal carrier (2), so that the slide member (36) is manually freely displaceable when the positioning means (37) are disengaged, and is displaceable on the longitudinal carrier (2) in a controlled manner **characterised by** fine adjustment and positive engagement when the positioning means (37) are engaged.

2. An apparatus as claimed in claim 1, **characterised in that** the first joints (8) have three rotative degrees of freedom.

3. An apparatus as claimed in claim 1 or 2, **characterised in that** the second joints (33) have three rotative degrees of freedom.

4. An apparatus as claimed in any of the claims 1 to 3, **characterised in that** each transverse coupler (5) is telescopable, and releasably lockable, along an axis of telescopic extension (9; 13) extending perpendicularly to the longitudinal axis (3).

5. An apparatus as claimed in any of the claims 1 to 4, **characterised in that** each transverse coupler (5) comprises a releasably lockable, third joint (45).

6. An apparatus as claimed in claim 5, **characterised in that** the first, second, and third joints (8; 33; 45) of each transverse coupler (5) are releasably lockable by means of a locking element (44).

7. An apparatus as claimed in claim 6, **characterised in that** the longitudinal carrier (2) has an external screw thread (38) and the slide member (36) comprises a sleeve (39) displaceable on the longitudinal carrier (2) parallel to the longitudinal axis (3), the positioning means (37) connected therewith and realised in the form of an axially separable, threaded sleeve, and operating levers (40) hingedly mounted in the sleeve (39), the threaded sleeve being radially spread apart, by actuating the operating levers (40), to such an extent that the internal screw thread of the threaded sleeve is no longer in engagement with the external screw thread (38).

8. An apparatus as claimed in any of the claims 1 to 7, **characterised in that** it comprises a positioner (6) which is provided with a transverse rod (41) having a central axis (11), a free end portion (42) for positioning and/or guiding surgical instruments relative to a bone, and a fixed end portion (43), the transverse rod (41) being connected with its fixed end portion (43) to the slide member (36) and being telescopable coaxially to the central axis (11).

9. A positioning apparatus as claimed in claim 8, **characterised in that** various different templates (7) for guiding surgical instruments or tools may be mounted on the free end portion (42) of the positioner (6).

10. A positioning apparatus as claimed in claim 8 or 9, **characterised in that** several templates (7) may be mounted simultaneously on the positioner (7).

11. A positioning apparatus as claimed in any of the claims 8 to 10, **characterised in that** the slide member (36) comprises drive means (51) by means of which the transverse rod (41) is rotatable about the central axis (11).

12. A positioning apparatus as claimed in any of the claims 8 to 11, **characterised in that** the slide member (36) comprises a drive element (10) by means of which the transverse rod (41) is rotatable about an axis of rotation (12) which extends perpendicularly to the longitudinal axis (3) and perpendicularly to the central axis (11).

13. A positioning apparatus as claimed in any of the claims 8 to 12, **characterised in that** the transverse rod (41) is telescopable using means for coarse and fine adjustment.

14. A positioning apparatus as claimed in any of the claims 11 to 13, **characterised in that** the drive means (51) comprise means for coarse and fine adjustment.

15. A positioning apparatus as claimed in any of the claims 12 to 14, **characterised in that** the drive element (10) comprises means for coarse and fine adjustment.

16. A positioning apparatus as claimed in any of the claims 13 to 15, **characterised in that** the means for coarse and fine adjustment are infinitely variable.

17. A positioning apparatus as claimed in any of the claims 13 to 15, **characterised in that** the means for coarse and fine adjustment are gradually variable.

18. A positioning apparatus as claimed in any of the claims 13 to 17, **characterised in that** the means for coarse and fine adjustment are embodied in such a way as to be retained by friction.

19. A positioning apparatus as claimed in any of the claims 13 to 17, **characterised in that** the means for coarse and fine adjustment are embodied in such a way as to be lockable.

20. A positioning apparatus as claimed in any of the claims 9 to 19, **characterised in that** the templates (7) comprise bores (16) for receiving bone screws.

21. An apparatus as claimed in any of the claims 1 to 20, **characterised in that** it comprises bone fixation devices (17) which are connectible to the front end portions (35).

22. An apparatus as claimed in claim 21, **characterised in that** the bone fixation devices (17) are each hingedly connected with one front end portion (35) by means of the second joints (33).

23. A positioning apparatus as claimed in any of the claims 9 to 22, **characterised in that** it comprises tracer elements (18) which are attachable to the template (7).

24. A positioning apparatus as claimed in any of the claims 21 to 23, **characterised in that** it comprises tracer elements (18) which are attachable to the bone fixation devices (17).

25. A positioning apparatus as claimed in claim 23 or 24, **characterised in that** each tracer element (18) comprises at least three non-linear, electromagnetically or acoustically effective markers (19) the position of which relative to a space-based coordinate system (22) may be detected by means of an electronic position detector (19).

## Revendications

1. Dispositif permettant de positionner et/ou de guider des instruments chirurgicaux, comportant
A) un support longitudinal (2) en forme de tige, lequel comprend un axe longitudinal (3), une première extrémité (30), une deuxième extrémité (31) et une partie centrale (32);
B) deux supports transversaux (5) disposés transversalement à l'axe longitudinal (3), lesquels présentent respectivement une extrémité arrière (34) et une extrémité avant (35) et qui peuvent être fixés, au niveau des extrémités avant (35), respectivement sur un ou deux os, ou sur un cadre porteur;
C) deux mâchoires (4;14) pouvant coulisser sur le support longitudinal (2) parallèlement à l'axe longitudinal (3) et être fixées de manière amovible sur ledit support, lesquelles sont destinées à relier les extrémités arrière (34) des supports transversaux (5) au support longitudinal (2); et
D) un chariot (36) pouvant coulisser axialement sur le support longitudinal (2) par rapport à l'axe longitudinal (3) et être bloqué sur ledit support,
E) chaque mâchoire (4;14) comprenant une première articulation (8) pouvant être bloquée de manière amovible et permettant de bouger le support transversal (5) correspondant par rapport à l'axe longitudinal (2) sur au moins deux axes situés perpendiculairement l'un à l'autre,
**caractérisé en ce que**
F) chaque support transversal (5) comprend une deuxième articulation (33) pouvant être bloquée de manière amovible; et
G) le chariot (36) présente des moyens de positionnement (37) pouvant s'encliqueter sur le support longitudinal (2) ou être décliquetés de celui-ci, de sorte qu'il est possible, lorsque les moyens de positionnement (37) sont décliquetés, de faire coulisser librement à la main le chariot (36) et, lorsque les moyens de positionnement (37) sont encliquetés, de le faire coulisser par conjugaison de forme et avec réglage fin sur le support longitudinal (2).

2. Dispositif selon la revendication 1, **caractérisé en ce que** les premières articulations (8) présentent trois degrés de liberté de rotation.

3. Dispositif selon la revendication 1 ou 2, **caractérisé en ce que** les deuxièmes articulations (33) présentent trois degrés de liberté de rotation.

4. Dispositif selon l'une des revendications 1 à 3, **caractérisé en ce que** chaque support transversal (5) peut être rallongé et raccourci de manière télescopique, et bloqué de manière amovible, le long d'un axe de rallongement et de raccourcissement télescopique (9;13) s'étendant transversalement à l'axe longitudinal (3).

5. Dispositif selon l'une des revendications 1 à 4, **caractérisé en ce que** chaque support transversal (5) comprend une troisième articulation (45) pouvant être bloquée de manière amovible.

6. Dispositif selon la revendication 5, **caractérisé en ce que**, pour chaque support transversal (5), les premières, deuxièmes et troisièmes articulations (8,33,45) peuvent être bloquées de manière amovible au moyen d'un élément de blocage (44).

7. Dispositif selon la revendication 6, **caractérisé en ce que** le support longitudinal (2) présente un filet extérieur (38) et **en ce que** le chariot (36) comprend un manchon (39) pouvant coulisser sur le support longitudinal (2) parallèlement à l'axe longitudinal (3), des moyens de positionnement (37) réalisés sous forme de douille filetée pouvant être divisée axialement et des leviers de manoeuvre (40) logés dans ledit manchon (39) de manière à pouvoir pivoter, la douille filetée pouvant être ouverte radialement de manière suffisante par actionnement des leviers de manoeuvre (40) pour que le filet intérieur de la douille filetée ne soit plus en prise avec le filet extérieur (38).

8. Dispositif selon l'une des revendications 1 à 7, **caractérisé en ce qu'**il comprend un élément de positionnement (6) qui présente une tige transversale (41) avec un axe central (11), une extrémité libre (42) permettant de positionner et/ou de guider des instruments chirurgicaux par rapport à un os et une extrémité fixe (43), la tige transversale (41) pouvant être reliée avec son extrémité fixe (43) au chariot (36) et pouvant être rallongée et raccourcie de manière télescopique coaxialement à l'axe central (11).

9. Dispositif de positionnement selon la revendication 8, **caractérisé en ce que** différents gabarits de guidage (7) permettant de guider des instruments ou des outils chirurgicaux peuvent être montés sur l'extrémité libre (42) de l'élément de positionnement (6).

10. Dispositif de positionnement selon la revendication 8 ou 9, **caractérisé en ce que** plusieurs gabarits de guidage (7) peuvent être montés simultanément sur l'élément de positionnement (6).

11. Dispositif de positionnement selon l'une des revendications 8 à 10, **caractérisé en ce que** le chariot (36) comprend des moyens d'entraînement (51 ) permettant à la tige transversale (41) de tourner sur l'axe central (11).

12. Dispositif de positionnement selon l'une des revendications 8 à 11, **caractérisé en ce que** le chariot (36) comprend un élément d'entraînement (10) permettant à la tige transversale (41) de tourner sur un axe de rotation (12) s'étendant perpendiculairement à l'axe longitudinal (3) et perpendiculairement à l'axe central (11).

13. Dispositif de positionnement selon l'une des revendications 8 à 12, **caractérisé en ce que** la tige transversale (41) peut être rallongée et raccourcie de manière télescopique à l'aide de moyens d'ajustage rapide et fin.

14. Dispositif de positionnement selon l'une des revendications 11 à 13, **caractérisé en ce que** les moyens d'entraînement (51) comprennent des moyens d'ajustage rapide et fin.

15. Dispositif de positionnement selon l'une des revendications 12 à 14, **caractérisé en ce que** l'élément d'entraînement (10) comprend des moyens d'ajustage rapide et fin.

16. Dispositif de positionnement selon l'une des revendications 13 à 15, **caractérisé en ce que** les moyens d'ajustage rapide et fin sont à ajustage continu.

17. Dispositif de positionnement selon l'une des revendications 13 à 15, **caractérisé en ce que** les moyens d'ajustage rapide et fin sont à ajustage gradué.

18. Dispositif de positionnement selon l'une des revendications 13 à 17, **caractérisé en ce que** les moyens d'ajustage rapide et fin sont réalisés de manière à pouvoir être autobloquants.

19. Dispositif de positionnement selon l'une des revendications 13 à 17, **caractérisé en ce que** les moyens d'ajustage rapide et fin sont réalisés de manière à pouvoir être bloqués.

20. Dispositif de positionnement selon l'une des revendications 9 à 19, **caractérisé en ce que** les gabarits de guidage (7) comprennent des trous (16) destinés à recevoir des vis d'ostéosynthèse.

21. Dispositif selon l'une des revendications 1 à 20, **caractérisé en ce qu'**il comprend des moyens de fixation d'ostéosynthèse (17) qui peuvent être reliés aux extrémités avant (35).

22. Dispositif selon la revendication 21, **caractérisé en ce que** les moyens de fixation d'ostéosynthèse (17) peuvent être respectivement reliés, au moyen des deuxièmes articulations (33), à une extrémité avant (35) de manière à pouvoir tourner.

23. Dispositif de positionnement selon l'une des revendications 9 à 22, **caractérisé en qu'**il comprend des éléments de marquage (18) qui peuvent être montés sur le gabarit de guidage (7).

24. Dispositif de positionnement selon l'une des revendications 21 à 23, **caractérisé en qu**'il comprend des éléments de marquage (18) qui peuvent être montés sur les moyens de fixation d'ostéosynthèse (17).

25. Dispositif de positionnement selon la revendication 23 ou 24, **caractérisé en ce que** chaque élément de marquage (18) comprend au moins trois marqueurs (19) à effet électromagnétique ou acoustique disposés de manière non-linéaire et dont la position peut être saisie par un dispositif de saisie de position électronique (19) par rapport à un système de coordonnées (22) fixe dans l'espace.
